# EUROPÄISCHE PATENTSCHRIFT

(11) **EP 0 535 567 B1**
(45) Veröffentlichungstag und Bekanntmachung des Hinweises auf die Patenterteilung: **30.08.1995**
(21) Anmeldenummer: 92116532.0
(22) Anmeldetag: 28.09.1992
(51) Int. Cl.: A61K 9/127

(54) **Flüchtige Inhalationsnarkotika enthaltende Liposomen, ihre Herstellung und Verwendung**
Liposomes containing volatile inhalation anaesthetics, their preparation and their use
Liposomes contenant des anéstésiques d'inhalation volatiles, leurs préparations et leurs utilisations

(30) Priorität: 01.10.1991 DE 4132677
(43) Veröffentlichungstag der Anmeldung: 07.04.1993
(73) Patentinhaber: B. Braun Melsungen AG, 34209 Melsungen (DE)
(72) Erfinder: Schmitt, Jürgen, Dr., W-6313 Homberg/Ohm 3 (DE); Nehne, Jörg, Dr., W-3501 Guxhagen (DE); Berger, Simone, W-3509 Malsfeld/Dagobertshausen (DE); Peter, Horst, W-3503 Lohfelden (DE)
(74) Vertreter: von Kreisler, Alek, Dipl.-Chem.

(56) Entgegenhaltungen:
- EP-A- 0 274 431
- EP-A- 0 488 142
- WO-A-85/00011
- WO-A-90/12565
- WO-A-91/07171
- BIOCHEMISTRY Bd. 26, Nr. 9, 5. Mai 1987, EASTON (US) Seiten 2449 - 2458 N.C.CRAIG ET AL. 'effects of halothane on dipalmitoylphosphatidylcholine liposomes:a raman spectroscopic study'

## Beschreibung

Die vorliegende Erfindung betrifft flüchtige Inhalationsnarkotika enthaltende Liposomen, ihre Herstellung und Verwendung.

Die Erfindung betrifft insbesondere ein Verfahren zur Verkapselung von leicht flüchtigen, flüssigen, wasserunlöslichen organischen Verbindungen, die unter anderem zur Inhalationsnarkose eingesetzt werden können, diese Verbindungen enthaltende Phospholipid-Liposomen aus einem Phospholipid oder Phospholipid-Gemisch, sowie die intravenöse Anwendung dieser Liposomen zur Erzeugung einer Narkose.

Liposomen sind von geschlossenen Lipid-Bilayern umgebene Kügelchen, die einen wäßrigen Kern besitzen. Sie wurden zuerst beschrieben von Bangham, A.D., et al (J. Mol. Biol. 13, (1965) S.238-252) und sind in den folgenden Jahren sehr eingehend untersucht worden.

Nach grundlegenden Arbeiten über die Herstellung und Charakterisierung von Liposomen rückten ihre pharmazeutisch und medizinisch nutzbaren Eigenschaften mehr in den Vordergrund des Interesses. Dabei steht besonders die Fähigkeit der Liposomen, sowohl wasserlösliche als auch lipidlösliche Wirkstoffe zu verkapseln und damit als Arzneimittel-Carrier zu fungieren im Vordergrund. Einen guten Überblick über diese Möglichkeiten zum Einsatz der Liposomen gibt Gregoriadis, G., et al. (Ann. N.Y. Acad. Sci. 446, (1985) S. 319-340). Liposomen können durch eine ganze Reihe unterschiedlicher Herstellungsmethoden erhalten werden. Eine Übersicht über Vor- und Nachteile der einzelnen Methoden geben Szoka, F., et al. (Ann. Rev. Biophys. Bioeng., (1980) 9, S. 467-508) und Gregoriadis, G. (Liposome Technology, Vol. 1, 2 und 3, CRC Press, Inc., Boca Raton, Florida (1984).

Die Liposomen selbst werden aufgrund ihrer unterschiedlichen Größe und Anzahl ihrer Lipidmembranen in die drei Gruppen "multilamellar vesicles" (MLV; 0,5 bis mehrere »m), "small unilamellar vesicles" (SUV; 0,02 bis 0,05 »m) und "large unilamellar vesicles" (LUV; 0,06 bis 0,5 »m) eingeteilt. Jede einzelne Liposomenart kann durch unterschiedliche Methoden hergestellt werden. Dabei sind natürlich nur einige wenige Methoden geeignet, Wirkstoff-beladene Liposomen herzustellen, die in Arzneimitteln zum Einsatz kommen können.

Zu den wichtigsten Methoden, um Liposomen in pharmazeutisch akzeptabler Qualität und Menge herzustellen, zählen u.a. unterschiedliche Hochdruck-Homogenisationsverfahren und verschiedene Extrusionstechniken.

Aufgrund der Vesikelstruktur der Liposomen und des amphiphilen Charakters ihrer Hauptbestandteile, der Phospholipide, können in ihnen sowohl wasserlösliche als auch wasserunlösliche Wirkstoffe eingeschlossen werden. Als Transportvehikel von lipophilen und hydrophilen Wirkstoffen werden die Liposomen in vielen Bereichen von Therapie und Diagnostik eingesetzt (Knight, E.G. ed., Research monographs in cell and tissue physiology, Vol. 7, Liposomes: From physical structure to therapeutic applications, Elsevier, North-Holland, Biomedical Press, Amsterdam, New York, Oxford 1981). Wegen der Möglichkeit, sehr viele unterschiedliche Wirkstoffe liposomal zu verkapseln, ist im Laufe der Zeit ein sehr breites Anwendungsspektrum entstanden, wobei der Schwerpunkt bei der parenteralen Anwendung (i.v., s.c., i.m., i.p.) liegt.

Folgende Vorteile lassen sich durch liposomale Verkapselung von Wirkstoffen gegenüber den herkömmlichen Zubereitungen erzielen:
a) Schutz des Wirkstoffes vor vorzeitigem Abbau (in vivo und in vitro)
b) Anlegen eines Wirkstoffdepots mit gezielter Wirkstofffreisetzung
c) Verminderung der Toxizität von Wirkstoffen
d) Erhöhung der Wirksamkeit bestimmter Wirkstoffe
e) spezifische Gewebeverteilung der wirksamen Bestandteile (natürliches "Zielorgan" RES)
f) Überführung lipophiler Wirkstoffe in eine wasserlösliche Form.

Für eine Übersicht dieser und weiterer Vorteile des Einsatzes wirkstoffbeladener Liposomen siehe: Mihalko, P.J., Schreier, H., Abra, R.M., Liposomes as Drug Carriers (G. Gregoriadis ed.), Wiley and Sons, New York 1988, insbesondere S. 679-694.

Die unter a) bis f) aufgeführten Vorteile liposomal verkapselter Wirkstoffe haben dazu geführt, daß in den letzten Jahren in den unterschiedlichsten Therapiebereichen wirkstoffhaltige Liposomen in Tierexperimenten und klinischen Untersuchungen eingesetzt worden sind. (Schenk, P., et al., Internationale Trends in der Entwicklung von Liposomen unter besonderer Berücksichtigung der Patentsituation, Pharmazie 43, (1988) S. 457-463; Fichtner, J. und Arndt, D., Stand und Perspektiven der Liposomenforschung, Pharmazie 44; (1989) S. 752-757).

Die US-A-4 708 861 betrifft Liposomgel-Zusammensetzungen, die ein biologisch aktives Mittel enthalten, wobei diese Liposomen in einer speziellen Gelmatrix vorliegen. Das biologische Mittel soll u.a. ein Betäubungsmittel (narcotic) sein und die Liposomgel-Zusammensetzung kann u.a. auch injiziert werden, wie sich aus den Ansprüchen 1, 46, 49 sowie Spalte 9, Zeilen 61-76 ergibt. Nach den Ausführungsbeispielen wird allerdings ausschließlich ein Hormon als Wirkstoff in diesen Zusammensetzungen parenteral, d.h. intramuskulär, subkutan oder intraperitoneal, verabreicht. Nach der Lehre der Druckschrift ist es der Zweck des Einschlusses der Liposomen in der Gelmatrix, einen Retard-Effekt zu erzielen bzw. ein Wirkstoff-Reservoir anzulegen, wie sich aus Spalte 1, Zeilen 47-68 ergibt. Wie die Ausführungsbeispiele zeigen, ist mit diesen Formulierungen auch keine intravenöse Applikation sinnvoll, schon gar nicht zur Durchführung einer allgemeinen Anästhesie. Wie dem Fachmann auf diesem Gebiet bekannt und wie sich beispielsweise aus Hawley's Condensed Chemical Dictionary, 11. Auflage, 1987, Stichwort "narcotic" auf Seite 810 ergibt, ist dieser Begriff wie folgt definiert:
"A natural, semisynthetic, or synthetic nitrogen-containing heterocyclic drug that characteristically effects sleep (coma) and relief of pain, but also may result in addiction, i.e. a biochemical situation in which the body tissues become so adapted to the drug that they can no longer fuction normally without it. Natural narcotics are the plant products morphine and codeine (constituents of opium), both of which are alkaloids. ... Semisynthetic narcotics are modifications of the morphine molecule, e.g. diacetylmorphine (heroin), ... . Synthetic narcotics are meperidine, ethadone and phenazocine."

Die US-A-4 721 612 betrifft ein Verfahren zur in-vivo-Applikation von mit Wirkstoff beladenen Liposomen, die als uni- oder multilamellare Bilayer ein Salz eines organischen Säurederivats, eines Sterols, vorzugsweise des Cholesterols, enthalten, d.h., als Liposom wird ein Sterol-Liposom eingesetzt. Der Wirkstoff kann u.a. ein Betäubungsmittel (narcotic) sein, das u.a. intravenös appliziert werden kann (Ansprüche 1, 14-16 sowie 41 und 47). Die Ausführungsbeispiele selbst beschreiben lediglich die Applikation von Indometazin und Diazepam enthaltenden Sterol-Liposomen.

Die WO 91/07171, veröffentlicht am 30.05.1991, beschreibt Liposphären mit einem hydrophoben Kern und einem Phospholipidmonolayer in Form einer Hülle, die weiterhin pharmazeutische Wirkstoffe, u.a. zur parenteralen Applikation, enthalten (Ansprüche 1, 2 und 7).

Die Entwicklungsschwerpunkte liegen allgemein auf dem Einsatz wirkstoffbeladener Liposomen in antineoplastischen, antimikrobiellen und antiviralen Therapien, wobei besonders intensiv die liposomale Verkapselung der folgenden, beispielhaft aufgeführten Wirkstoffe untersucht worden ist:
Doxorubicin (US-A-4 460 577; Sculier, J.P., et al., Eur. J. Cancer Clin. 24, S. 527-538, 1988), Gentamycin (WO 88/04573; EP 0 153 364; Morgan, J.R., Williams, K.E., Antimicrobial Agents and Chemotherapy 17, S. 544-548, 1980; Swenson, Ch., E., ibid. 34, S. 235-240, 1990); Adriamycin (DE-OS 28 42 608); Amphotericin B (Lopez-Berestein, G., Ann. Intern. Med. 105, S. 130-131, 1986), Interleukin 2 (GB-A-2 157 172; Anderson, P.M. et al., Cancer Res. 50, 1853-1856 (1990)), und Na-Stibogluconat (EP-A-0 072 234; Carter et al., Liposomes in the therapy of infectious diseases and cancer, Lopez-Berestein, G., Fidler, I.J. edts., S. 215-226, Alan R. Liss, Inc., 1989). Des weiteren ist außerdem der liposomale Einschluß einer großen Anzahl von Substanzen, z.B. aus den Gruppen der Kontrastmittel, Antiphlogistica, Antiparasitica und Proteine/Peptide untersucht und in der einschlägigen Literatur beschrieben.

Bei allen diesen oben erwähnten liposomalen Formulierungen ist mindestens einer der Punkte a) bis f), im Vergleich mit herkömmlichen galenischen Zubereitungen erfüllt. Wobei vor allem bei den liposomal verkapselten Antiparasitica die deutliche Verringerung der Toxizität gegenüber dem freien Wirkstoff und die gezielte Therapie innerhalb des RES von Bedeutung sind.

Sollen Wirkstoffe parenteral (i.v., s.c., i.m., i.p.) verabreicht werden, ist es notwendig, daß die Substanzen in wasserlöslicher Form oder zumindest in einer wäßrigen Suspension verfügbar sind. Dies gilt vor allen Dingen, wenn eine intravenöse Applikation zur Entfaltung der Wirksamkeit des Arzneimittels unerläßlich ist. Problematisch sind deshalb lipophile Wirkstoffe, die in eine wasserlösliche Form überführt werden sollen. Grundsätzlich bieten sich dazu mehrere unterschiedliche galenische Möglichkeiten an, z.B.:
1. Einsatz von Lösungsvermittlern (z.B. Cremophor^{R} EL) (Techniques of Solubilizing Drugs, S.H. Yalkowski ed., 1981, Marcel Dekker, New York, S. 15-134).
2. Fettemulsionen (Parenteral Emulsions for Drug Delivery, L.C. Collins- Gold, R.T. Lyons, L.C. Bartholow, Advanced Drug Delivery Reviews 5, S. 189-208 (1990)).
3. Sogenannte "Microdroplets". (Nach D.C. Haynes et al. (1985) Anesthesiology 63, S. 490-499 werden die Microdroplets erhalten, indem Lecithin und das Inhalationsanästhetikum Methoxyfluran in einem wäßrigen Träger mit einer Polytronvorrichtung suspendiert werden, dann diese Suspension ultrabeschallt wird um Microdroplets zu erhalten; daraufhin wird die Reaktionsmischung bei etwa 4000 g zentrifugiert um größere Partikel zu entfernen, dann wird eine Ultrafiltration durchgeführt, um Teilchen größer 450 nm zu entfernen und schließlich die in Ampullen abgefüllten Microdroplets sterilisiert. Die so erhaltenen Microdroplets weisen eine Teilchengröße von etwa 100 nm auf. Ein ähnliches Herstellungsverfahren für Mikrodroplets ist Gegenstand der WO 85/00011.)
4. Einschlußverbindungen, z.B. mit Hilfe von Cyclodextrinderivaten (Duchene, D., Wouessidjew, D. (1990) Pharm. Technol. Int. 6, 21-29; Brewster, M.E. et al. (1990) Int. J. Pharm. 59, 231-243; EP 0 149 197).
5. Liposomen.

Zu den Punkten 1. und 3. existieren sehr viele unterschiedliche Bezeichnungen (z.B. micellare Lösungen, Nanoemulsionen, Micropheres, etc.), die sich begrifflich und auch aus galenischer Sicht nur schwer gegeneinander abgrenzen lassen, deshalb sollen sie alle unter den beiden Oberbegriffen zusammengefaßt werden.

Der vorliegenden Erfindung liegt die Aufgabe zugrunde, flüchtige Inhalationsnarkotika enthaltende Liposome bereitzustellen. Weitere Aufgabe der vorliegenden Erfindung ist es, ein Herstellungsverfahren für diese Liposomen bereitzustellen. Schließlich beabsichtigt die vorliegende Erfindung, diese Liposomen in der Anästhesie intravenös einzusetzen.

Gegenstand der vorliegenden Erfindung sind daher flüchtige Inhalationsnarkotika enthaltende Liposome, erhältlich durch
- Vordispergieren von 1 bis 40 Gew.-%, vorzugsweise 5 bis 20 Gew.-% wenigstens eines Phospholipids,
- 0,01 bis 10 Gew.-%, vorzugsweise 1 bis 5 Gew.-% wenigstens eines Inhalationsnarkotikums und
- 50 bis 98,99 Gew.-%, vorzugsweise 75 bis 94 Gew.-% eines Hydratisierungsmediums unter Bildung einer überwiegend multilamellare Vesikel enthaltenden Lipid-Wirkstoffdispersion,
- Extrudieren oder Homogenisieren unter Bildung überwiegend unilamellare Inhalationsnarkotika enthaltender Liposome und
- Sterilfiltern in an sich bekannter Weise,
die vorzugsweise 0 bis 50 Gew.-Teile, vorzugsweise 1 bis 2,5 Gew.-Teile üblicher Hilfs- und Zusatzstoffe pro 100 Gew.-Teile Ausgangslösung aus Phospholipid, Inhalationsnarkotikum und Hydratisierungsmedium enthalten.

Weiter betrifft die Erfindung ein Verfahren zur Herstellung von flüchtige Inhalationsnarkotika enthaltenden Liposomen durch
- Vordispergieren von 1 bis 40 Gew.-%, vorzugsweise 5 bis 20 Gew.-% wenigstens eines Phospholipids,
- 0,01 bis 10 Gew.-%, vorzugsweise 1 bis 5 Gew.-% wenigstens eines Inhalationsnarkotikums und
- 50 bis 98,99 Gew.-%, vorzugsweise 75 bis 94 Gew.-% eines Hydratisierungsmediums unter Bildung einer überwiegend multilamellare Vesikel enthaltenden Lipid-Wirkstoffdispersion,
- Extrudieren oder Homogenisieren unter Bildung von überwiegend unilamellaren Inhalationsnarkotika enthaltenden Liposomen und
- anschließende Sterilfiltration in an sich bekannter Weise,
die vorzugsweise 0 bis 50 Gew.-Teile, vorzugsweise 1 bis 2,5 Gew.-Teile üblicher Hilfs- und Zusatzstoffe pro 100 Gew.-Teile Ausgangslösung aus Phospholipid, Inhalationsnarkotikum und Hydratisierungsmedium enthalten.

Schließlich betrifft die vorliegende Erfindug die Verwendung der vorstehenden Liposomen bzw. der nach vorstehendem Verfahren hergestellten Liposomen zur Allgemeinanästhesie durch intravenöse Applikation, auch in Kombination mit anderen üblichen intravenösen Narkosemitteln.

Vorzugsweise handelt es sich bei dem Phospholipid um ein einzelnes Phospholipid oder ein Phospholipidgemisch, das überwiegend aus Phospholipiden besteht, welches ausgewählt ist aus natürlichen, pflanzlichen oder tierischen Phospholipiden, halbsynthetischen oder synthetischen Phospholipiden. Vorzugsweise handelt es sich bei dem Inhalationsnarkotikum um einen fluorhaltigen Kohlenwasserstoff oder Ether mit einem Siedepunkt von 20 °C bis 105 °C, vorzugsweise 48 °C bis 105 °C. Besonders bevorzugt sind die Inhalationsnarkotika, die ausgewählt sind aus den Wirkstoffen Halothan, Methoxyfluoran, Enfluran, Isofluran, Desfluran und/oder Sevofluran. Vorzugsweise setzt man als Hydratisierungsmedium eine physiologisch verträgliche isotone wäßrige Lösung ein.

Die vorliegende Erfindung beschreibt u.a. den Einsatz von Liposomen zur Verkapselung von lipophilen, wasserunlöslichen Wirkstoffen. Durch ihren strukturellen Aufbau (Bilayermembran) und ihre Definition sind die Liposomen deutlich gegenüber den anderen oben aufgeführten galenischen Zubereitungsformen abzugrenzen. Die Liposomen haben gegenüber den Zubereitungen 1 bis 3 unter anderem die folgenden Vorteile, die sie für die in der vorliegenden Erfindung beabsichtigte Verwendung besonders bevorzugt erscheinen lassen:
1. Es kommen keine organischen Lösungsmittel bei der Herstellung der Liposomen nach dem erfindungsgemäßen Verfahren zum Einsatz.
2. Eventuell toxische Detergentien oder Cosolventien werden vermieden.
3. Einsatz von gut verträglichen und toxikologisch unbedenklichen Rohstoffen (Phospholipide natürlichen Ursprungs) zur Solubilisierung der Wirkstoffe.
4. Stabile Verkapselung der entsprechenden Wirkstoffe.
5. Dem Charakter der Wirkstoffe (leicht flüchtig, niedrig siedend) entsprechende Sterilisationsmöglichkeit (Sterilfiltration).
6. Eignung für eine intravenöse Injektion/Infusion.

In der vorliegenden Erfindung wird ein Verfahren zur liposomalen Verkapselung von lipophilen, flüssigen und leicht flüchtigen organischen Verbindungen und die Verwendung der daraus resultierenden wirkstoffhaltigen Liposomen zur Durchführung einer Allgemeinanästhesie durch intravenöse Injektion/Infusion beschrieben.

Zur Durchführung einer Allgemeinanästhesie über einen längeren Zeitraum und bei größeren medizinischen Eingriffen wird üblicherweise nach der Narkoseeinleitung durch ein Kurzzeitnarkotikum, wenn möglich mit einer analgetischen Komponente (z.B. Fentanyl, Ketamin) die Narkoseführung mit Inhalationsnarkotika vorgenommen (C.C. Alpert et al. (1985) Clin. Plast. Surg. 12, 33-42). Typische Inhalationsnarkotika sind unter anderem Methoxyfluran, Enfluran, Isofluran und Halothan. Kennzeichnend für diese Substanzen ist ein hoher Dampfdruck, der ausgenutzt wird, um die Stoffe über einen Verdampfer dem zur künstlichen Beatmung der Patienten dienenden Gasgemisch (Sauerstoff/Lachgas) beizugeben.

Neben der Inhalationsnarkose wird auch, allerdings noch in etwas geringerer Häufigkeit, die intravenöse Narkose zur Allgemeinanästhesie eingesetzt. Im Gegensatz zu den Substanzen, die bei der Inhalationsnarkose benutzt werden, stammen die intravenösen Narkosemittel aus unterschiedlichen Stoffgruppen (z.B. Etomidat, Thiopental).

Bei den Inhalationsnarkotika erfolgt die Wirkungsbeendigung durch Abatmung des im Blut befindlichen Wirkstoffes, dagegen bei den intravenösen Narkosemitteln durch einen für jeden Wirkstoff spezifischen Abbaumechanismus. Einen guten Überblick über die Indikationen und allgemeinen Wirkungsweisen der Inhalationsnarkotika und intravenösen Narkosemittel erhält man in: Hossli, G., Jenny, R., Grundlagen 2 der Anästhesiologie, Verlag Hans Huber, Bern, Stuttgart, 1987, und Lee, J.A., Atkinson, R.S., Synopsis der Anästhesie, Gustav Fischer Verlag, Stuttgart, 1978.

Die heute überwiegend eingesetzten Inhalationsnarkotika Enfluran und Isofluran haben gegenüber den früher häufiger benutzten Wirkstoffen, z.B. Methoxyfluran, Halothan und den meisten intravenösen Narkosemitteln, den Vorteil, daß sie aufgrund ihrer chemischen Stabilität nur zu einem sehr geringen Teil (Isofluran < 2 %) verstoffwechselt werden und zu unverträglichen oder auch toxischen Metaboliten führen.

Beide Narkoseverfahren (Inhalationsnarkose und intravenöse Narkose) haben natürlicherweise Vor- und Nachteile, die u.a. auf der Technik, bzw. auf den bei dem jeweiligen Verfahren verwendeten Narkosemitteln beruhen. So sind z.B. die gute Steuerbarkeit (Technik und Narkosemittel) und die minimale Belastung des Stoffwechsels (Narkosemittel) Vorteile der Inhalationsnarkose, dagegen sind die Schleimhautreizungen (Narkosemittel), unangenehmer Geruch (Narkosemittel), Umweltbelastung (Narkosemittel), Beatmungszwang (Technik und Narkosemittel), die fehlende Möglichkeit einer ambulanten Narkose (Technik), Nachteile dieses Narkoseverfahrens. Die Vorteile der intravenösen Narkose sind die für den Patienten angenehme Einleitung (Technik) und der rasche Wirkungseintritt (Technik und Narkosemittel). Die Nachteile der intravenösen Narkose liegen ausschließlich in den Eigenschaften der einzelnen Narkosemittel begründet. So ist z.B. die Wirkungsdauer von der Umverteilung, dem Abbau bzw. der Ausscheidung der eingesetzten Substanzen abhängig. Nebenreaktionen bei intravenös verabreichten Narkosemitteln sind des öfteren Myokard- und Atemdepressionen.

Die Verwendung des einen oder des anderen Narkoseverfahrens kann nach Art des medizinischen Eingriffs und Allgemeinzustand des Patienten indiziert sein. Die bereits oben zitierten anästhesiologischen Übersichtswerke geben hierüber detailliert Auskunft. Im allgemeinen werden beide Narkoseverfahren auch gleichzeitig benutzt ("balanced anesthesia"), d.h. Narkoseeinleitung intravenös, darauf Narkoseführung durch Inhalation.

Bei speziellen Indikationsgebieten (z.B. Komplikationen im Bereich der Atmungsorgane) wird sowohl die Narkoseeinleitung als auch die Narkoseführung durch intravenöse Narkose-Verfahren durchgeführt (total intravenous anesthesia). Es gibt aber auch Bestrebungen unter den Anästhesisten, das Verfahren der TIVA aufgrund der sehr positiven Erfahrungen auf weitere Indikationsgebiete auszudehnen.

In der vorliegenden Erfindung wird nun ein Verfahren zur Überführung von wasserunlöslichen, leicht flüchtigen, organischen Flüssigkeiten, die vorzugsweise zur Inhalationsnarkose eingesetzt werden, in eine intravenös applizierbare Form beschrieben. Die auf diese Weise erhaltenen Narkosemittel können zur Durchführung von intravenösen Narkosen eingesetzt werden. Die der Erfindung zugrundeliegende Idee ist, die oben dargestellten vorteilhaften stofflichen Eigenschaften der Inhalationsnarkotika, insbesondere Enfluran und Isofluran, auch für die intravenöse Narkose zugänglich zu machen.

Zusätzlich ergeben sich durch die Möglichkeit zur Durchführung einer intravenösen Narkose mit Inhalationsnarkotika noch folgende Vorteile:
1. Es ist kein Einleitungshypnotikum nötig.
2. Die Beatmung der Patienten kann mit Raumluft durchgeführt werden.
3. Durchführung einer "total intravenous anesthesia" wird erleichtert.
4. Bedingt durch das rasche Abfluten der Inhalationsnarkotika kommt es zu einer schnelleren Erhohlung des Patienten.
5. Dadurch, daß das Narkotikum sofort im Blutkreislauf ist, wird weniger Wirkstoff zur Durchführung einer Narkose benötigt (geringere Belastung der Umwelt und des Klinikpersonals).
6. Der apparative Aufwand zur Durchführung einer Narkose wird verringert.
7. Die erfindungsgemäßen Narkosemittel sind gut geeignet zur Durchführung einer ambulanten Anästhesie.

Angesichts einer Vielzahl von Vorteilen für intravenös applizierbare Inhalationsnarkotika wurden schon verschiedene Versuche unternommen, die entsprechenden Substanzen zur intravenösen Narkose einzusetzen.

Dabei wurden im wesentlichen drei Konzepte verfolgt:
A) Einarbeitung des Inhalationsnarkotikums (Methoxyfluran) in eine O/W-Emulsion in Gegenwart des synthetischen Emulgators Pluronic F68 (Krantz, J.C. et al. (1961) Anesthesiology 22, 431; Krantz, J.C. et al. (1962) Anesthesia and Analgesie 41, S. 257-262; Cascorbi, H.F. et al. (1968) Anesthesia and Analgesie, 47, S. 557-559).
B) Lösung des Inhalationsnarkotikums (Halothan) in handelsüblichen Fettemulsionen zur parenteralen Ernährung. (Johannesson, G. et al. (1984) Acta Anaesthesiol. Scand. 28, S. 381-384 beschreiben die intravenöse Verabreichung von Halothan in einer Sojaöl-Fettemulsion mit Eiphosphatid als Emulgator; Biber, B. et al. (1984) Acta Anaesthesiol. Scand. 28, S. 385-389 beschreiben die intravenöse Verabreichung von Halothan, gelöst in einer handelsüblichen Fettemulsion zur parenteralen Ernährung).
C) Intravenöse Applikation des reinen Inhalationsnarkotikums (Halothan, Methoxyfluran) (Hilwig, R.W. (1976) Am. J. Vet. Res. 37, S. 257-262; Dwyer, R., Coppel, D.L. (1989) Anesth. Analg. 69, S. 250-255).

Bei allen drei Konzepten führte die intravenöse Applikation des Inhalationsnarkotikums zwar zu einer Narkose, aber eine Realisierung und Nutzbarmachung der Konzepte für die klinische Anwendung scheiterte an galenischen Problemen und an Unverträglichkeiten, die z.T: auch durch die eingesetzten Wirkstoffe (Halothan, Methoxyfluran) hervorgerufen wurden. Deshalb wurden diese Entwicklungen nicht weitergeführt.

Im Falle der Emulsionen (Konzept A und B) ist besonders das Verbleiben des Wirkstoffes in der Ölphase zweifelhaft, vor allen Dingen, wenn man berücksichtigt, daß Emulsionen generell nur durch eine Hitzesterilisation einer intravenösen Applikation verfügbar gemacht werden können. Außerdem ist natürlich auch durch das Lösen des Wirkstoffs in einer Emulsion (Konzept B) keine Formulierung in gleichbleibender und reproduzierbarer Qualität und Wirksamkeit zu erhalten. Die Untersuchungen, die unter Punkt C aufgeführt sind, sind lediglich von wissenschaftlichem Interesse und haben keine klinische Relevanz.

In der neueren Literatur wird eine weitere galenische Alternative beschrieben, um Inhalationsnarkotika in einer Wasserdispergierbaren Formulierung zu erhalten. Es werden sog. "Microdroplets" gebildet, emulsionsartige Partikel, die, im Gegensatz zu Liposomen (Phospholipid-Bilayer, wäßriger Kern), von einem Phospholipid-Monolayer umgeben sind, aber kein Öl, sondern das Inhalationsnarkotikum bzw. ein organisches Lösungsmittel in ihrem Kern enthalten (D.C. Haynes et al. (1985) Anesthesiology 63, S. 490-499; M.J. Wu et al. (1989) J. Contr. Rel. 9, S. 1-12; WO 85/00011, entsprechend US-A-4 622 219). Da es sich bei diesen "Microdroplets" im eigentlichen Sinne um eine O/W-Emulsion handelt, gelten dafür natürlich die gleichen Einschränkungen wie bereits oben erwähnt. Außerdem werden diese "Microdroplets" zur Erzeugung einer Lokalanästhesie nach intramuskulärer Applikation verwendet.

In der vorliegenden Erfindung werden, abweichend von den oben erwähnten Untersuchungen, Phospholipid-Liposomen zur Solubilisierung der Inhalationsnarkotika eingesetzt. Die Liposomen haben den Vorteil, daß sie mit einer ganzen Reihe unterschiedlicher Methoden hergestellt werden können und die Teilchengröße so eingestellt werden kann, daß die wirkstoffhaltigen Suspensionen mit etablierten Methoden sterilfiltriert werden können. Außerdem entspricht die intravenöse Applikation von wirkstoffhaltigen Liposomen dem heutigen Stand der Technik (P.J. Mihalko et al. (1988) Liposomes as Drug Carriers, wie oben zitiert).

Bei der erfindungsgemäßen liposomalen Verkapselung von Inhalationsnarkotika müssen entsprechend den physikalisch-chemischen Eigenschaften dieser Verbindungen (Siedepunkte ab etwa 23,5 °C, vorzugsweise um 50 °C, leicht flüchtig) geeignete Maßnahmen, wie z.B. Kühlung, Schutzgas, Arbeiten unter einem Abzug, getroffen werden, um den Wirkstoffverlust und eine Gefährdung des Personals während der Herstellung so gering wie möglich zu halten. Prinzipiell kommen deshalb auch nur solche Herstellungsverfahren in Frage, bei denen die oben aufgeführten notwendigen Hilfsmaßnahmen getroffen werden können. Es eignen sich also zur Herstellung von Inhalationsnarkotika-haltigen Liposomen besonders die gängigen Extrusions- und Homogenisierungstechniken, auf die in den Beispielen näher eingegangen wird.

Die Herstellung der erfindungsgemäßen Liposomen geschieht in zwei voneinander unabhängigen Schritten, wobei im ersten Schritt, der sogenannten Vordispergierung, d.h. Bildung einer überwiegend multilamellare Vesikel enthaltenden Lipid-Wirkstoff-Dispersion, Phospholipide, eventuell benötigte andere Hilfsstoffe und das Inhalationsnarkotikum in einem geeigneten Hydratisierungsmedium dispergiert werden. In einem zweiten sich anschließenden Schritt werden dann die überwiegend unilamellaren Liposomen durch die geeigeneten Extrusions- oder Homogenisierungsverfahren gebildet. Nach durchgeführter Sterilfiltration können die Inhalationsnarkotika-haltigen Liposomendispersionen intravenös infundiert oder injiziert werden. Als besonders vorteilhaft hat sich herausgestellt, wenn während des Vordispergierungsschrittes unter Kühlung (< 4 °C), in einem gasdichten, abgeschlossenen Gefäß und unter Schutzgasüberdruck (z.B. Argon) gearbeitet wird (siehe Beispiele).

Die Vordispergierung kann auf zwei unterschiedliche Weisen vorgenommen werden:
a) Die Phospholipide und die eventuell benötigten Hilfsstoffe werden zuerst in dem Inhalationsnarkotikum aufgelöst und dann im Hydratisierungsmedium dispergiert (Beispiele 2, 4).
b) Phospholipide und Hilfsstoffe werden im Hydratisierungsmedium dispergiert und darauf erfolgt die Zugabe, am besten portionsweise, des Inhalationsnarkotikums (Beispiele 1, 3).

Die anschließend, mittels geeigneter Herstellungsverfahren gebildete, überwiegend unilamellare Liposomen enthaltende Dispersion wird mit gängigen Analysenmethoden hinsichtlich ihres strukturellen Aufbaus (Elektronenmikroskopie), Teilchengröße, Polydispersität (Photonenkorrelationsspektroskopie), Wirkstoffgehaltes (Gaschromatographie) und Stoffzusammensetzung (Dünnschichtchromatographie, Phospholipidbestimmung) analysiert.

Bei den der Erfindung zugrundeliegenden Verfahren zur liposomalen Verkapselung von Inhalationsnarkotika können prinzipiell alle üblicherweise zur Liposomenherstellung eingesetzten Phospholipide oder Phospholipid-Gemische benutzt werden. Im einzelnen können natürliche, halbsynthetische oder synthetische Phospholipide und/oder Phospholipid-Gemische eingesetzt werden.

Als Phospholipide und Phospholipid-Gemische natürlichen Ursprungs kommen vor allem die entsprechenden Substanzen aus Soja oder Ei in Frage. Dies sind z.B. Phospholipid-Gemische unterschiedlicher Zusammensetzung aus Phosphatidylcholin und Phosphatidylethanolamin mit einem geringen Anteil anderer Lipide oder Lipidklassen, wie z.B. neutrale Lipide, Glykolipide und Sphingomyeline.

Weitere einsetzbare Phospholipide aus anderen natürlichen, biologischen Materialien sind Phosphatidylcholin (PC), Phosphatidylethanolamin (PE), Phosphatidylglycerol (PG), Phosphatidylinosit (PJ), Phosphatidylserin (PS), Phosphatidsäure (PA).

Die vorstehend aufgeführten Lipide können des weiteren durch katalytische Hydrierung chemisch modifiziert werden und in unterschiedlichen Hydrierungsgraden Verwendung finden.

Synthetische und halbsynthetische Phospholipide mit definierter Fettsäurezusammensetzung, wie z.B. Di-palmitoyl-phosphatidylcholin (DPPC), Di-stearoyl-phosphatidylcholin (DSPC), Di-palmitoyl-phosphatidylethanolamin (DPPE), Di-stearoyl-phosphatidylethanolamin (DSPE), Di-myristoyl-phosphatidylserin (DMPS), Dioleyl-phosphatidylcholin (DOPC) und alle physikalisch möglichen Mischungen der einzelnen Substanzen.

Zusätzlich zu den oben aufgeführten Phospholipiden können zur Herstellung von Inhalationsnarkotika-haltigen Liposomendispersionen auch andere Lipide und lipidartige Substanzen als Hilfsstoffe eingesetzt werden. Dazu gehören z.B. C₄-C₂₀-Fettsäure-C₁-C₄-alkylester, die sich in das Liposomenbilayer einbauen lassen, z.B. Ölsäure- und Linolsäureethylester, und/oder C₄-C₂₀-Alkyl- oder Alken(poly)ylcarbonsäuren, bzw. deren Salze, wie z.B. Natriumoleat, und/oder die als Antioxidantien wirkenden Tocopherole, Tocopherolester und Ascorbylpalmitat, und/oder Stearylamin und Dicetylphosphat, die unter anderem zur Erzeugung einer Oberflächenladung auf den Liposomen eingesetzt werden können und/oder Verbindungen zur Stabilisierung der Bilayer, wie z.B. Cholesterin oder dessen Derivate. Als übliche Hilfs- und Zusatzstoffe werden lipidartige Substanzen in Mengen von 0 bis 50 Gew.-%, bezogen auf die Menge Phospholipid und/oder Antioxidantien in Mengen von 0,1 bis 1,0 mol-%, bezogen auf mol Phospholipid und/oder Stoffe zur Stabilisierung der Bilayer in Mengen von 0 bis 50 Gew.-%, bezogen auf die Menge Phospholipid, einsetzt.

Als wäßrige Phase bzw. Hydratisierungsmedien können alle zur Erzeugung einer physiologisch verträglichen, isotonischen Lösung üblicherweise eingesetzten Substanzen benutzte werden, z.B. physiologische Kochsalzlösung (0,9 %ige wäßrige Kochsalzlösung), 2,5 %ige Glycerinlösung, 5 %ige Xylitlösung. Um einen pH-Wert im physiologisch verträglichen Bereich zu erzeugen, können auch die entsprechenden Puffersubstanzen zugesetzt werden, so z.B. Tris-(hydroxymethyl)-aminomethan. Natürlich können für diese Zwecke auch alle anderen dem Stand der Technik entsprechenden Hilfsstoffe eingesetzt werden. Eine Übersicht darüber gibt Sucker, H. et al. (1991), Pharmazeutische Technologie, Georg Thieme Verlag, Stuttgart, New York, S. 174-216 u. S. 454-569.

Mit den in der Erfindung beschriebenen Verfahren können natürlich eine ganze Reihe von Wirkstoffen und Wirkstoffgruppen liposomal verkapselt werden, die oben bereits angedeuteten und in den nachfolgenden Beispielen näher ausgeführten Maßnahmen zum Arbeiten unter Kühlbedingungen sind aber notwendig, um die vorzugsweise in der Erfindung beschriebenen Inhalationsnarkotikahaltigen Liposomen herzustellen. Gleiches gilt natürlich auch, wenn Wirkstoffe eingesetzt werden sollten, die den Inhalationsnarkotika ähnliche chemisch-physikalische Eigenschaften haben.

Aus der Gruppe der Inhalationsnarkotika bzw. Substanzen mit ähnlicher chemischer Struktur und gleicher narkotischer Wirksamkeit (z.B. Diethylether, Chloroform) sind aus Sicht der erfindungsgemäßen Anwendung der wirkstoffhaltigen Liposomen zu einer intravenösen Narkose natürlich Wirkstoffe bevorzugt, die auch aus medizinischer Sicht unbedenklich sind und auch schon zur Durchführung von Inhalationsnarkosen eingesetzt worden sind. Dies sind hauptsächlich die folgenden Wirkstoffe:
1) Halothan: 2-Brom-2-chlor-1,1,1-trifluorethan
2) Methoxyfluran: (2,2-Dichlor-1,1-difluorethyl)methyl-ether
3) Enfluran: 2-Chlor-1,1,2-trifluorethyl(difluormethyl)ether
4) Isofluran: 1-Chlor-2,2,2-trifluorethyl(difluormethyl)ether

In jüngster Zeit sind noch zwei weitere Verbindungen mit ähnlichen chemisch-physikalischen Eigenschaften auf ihre narkotische Wirksamkeit hin untersucht worden (R.M. Jones (1990) Brit. J. Anaesth. 65, S. 527-536):
5) Desfluran: 2-Difluormethoxy-1,1,1-2-tetrafluorethan
6) Sevofluran: 1,1,1,3,3,3-Hexafluor-2-(fluormethoxy)-propan

Die vorliegende Erfindung betrifft deshalb insbesondere die liposomale Verkapselung der oben aufgeführten Wirkstoffe (1-4) sowie die Verwendung der entstehenden wirkstoffhaltigen Liposomen zur Durchführung einer intravenösen Allgemeinanästhesie.

Die nachfolgenden Beispiele dienen zur näheren Erläuterung der Erfindung.

Im folgenden wird unter der Abkürzung "KG" Körpergewicht verstanden. Die Bestimmung der Teilchengröße und der Polydispersität wird mittels Photonenkorrelationsspektroskopie (Cummins, P.G., Staples, E.J. (1987), LANGMUIR 3, 1109-1113) durchgeführt (Autosizer II_{c}, Fa. Malvern, England). Der Gehalt an Inhalationsnarkotika in der Liposomendispersion wurde mittels Headspace Gaschromatographie bestimmt. Bedingungen: Gaschromatograph Sigma 2000 mit Flammenionisationsdetektor (Fa. Perkin, Elmer), Trennsäule DB-1 (Fa. J & W Scientific), Trägergas Helium, Temperaturgradient, Detektortemperatur 280 °C.

### Beispiel 1

### Herstellung von Isofluran-haltigen Liposomen

### a) Vordispergierung

40 g eines Ei-Phospholipid-Gemisches (Hauptkomponenten: PC 70 bis 95 %, PE 5 bis 20 %, Phasenübergangstemperatur ca. -20 °C) werden in einem auf 4 °C vorgekühlten und mit Stickstoff begasten Laborreaktor (Innenvolumen: 1000 ml, Fa. IKA, Janke & Kunkel) eingewogen. Es werden etwa 150 ml auf 4 °C vorgekühltes Hydratisierungsmedium (10 mmol Tris HCl in 2,5 %iger wäßriger Glycerinlösung, pH 7,5) zugegeben, erneut mit Stickstoff begast und der Laborreaktor verschlossen. Daraufhin wird 15 min. lang gleichzeitig mit einem Ultraturrax (8000 U/min) und einem Rührer (20 U/min) und permanenter Kühlung (4 °C) behandelt. Anschließend werden 3 g Linolsäureethylester zu der Dispersion gegeben und unter identischen Bedingungen 15 min. lang weiterbehandelt. Dann werden 10 g Isofluran, vorgekühlt auf 4 °C, in drei Portionen zugegeben, der Laborreaktor mit Stickstoff begast und immer wieder gut verschlossen. Nach Beendigung der Isofluranzugabe wird noch 1 bis 2 min. weiterbehandelt. Im Anschluß daran wird mit gekühltem Hydratisierungsmedium auf ein Endvolumen von 200 ml aufgefüllt.

### b) Homogenisierung

Die unter 1a) hergestellte Vordispersion wird in einem Microfluidizier Y-100 (Fa. Microfluidics, USA) eingefüllt und 60 min. bei 750 bar und permanenter Kühlung (4 °C) homogenisiert. Die daraus resultierende Liposomendispersion wird durch einen 0,22 »m Sterilfilter filtriert und in Glasvials abgefüllt.

Die Analytik der Liposomendispersion ergibt folgende Ergebnisse:

| | |
|---|---|
| Aussehen, Färbung: | opaleszent, gelblich |
| Teichengröße: | 101,0 nm |
| Polydispersität: | 0,412 |
| Isoflurangehalt: | 31,2 mg/ml |

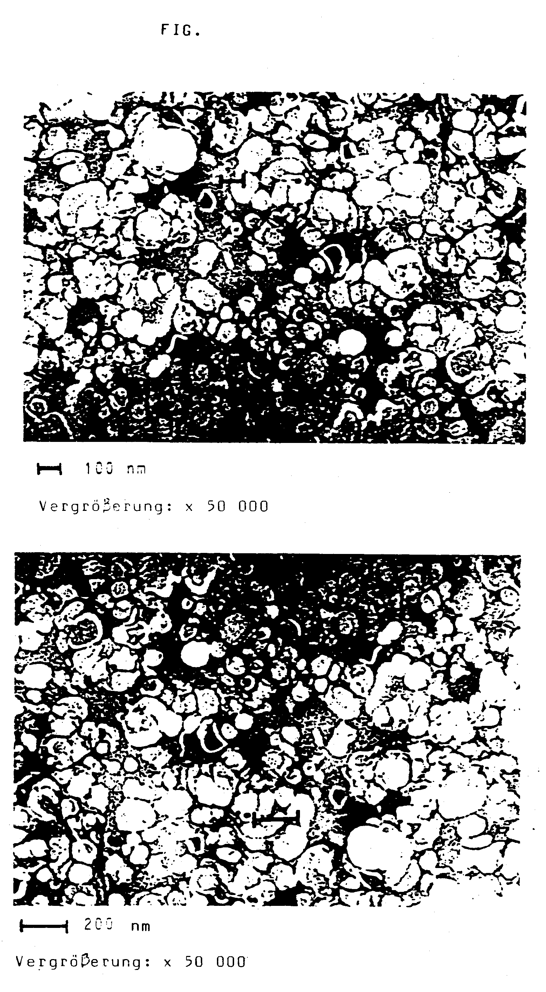

Die Fig. zeigt eine Photographie einer elektronenmikroskopischen Aufnahme von Liposomen, die gemäß Beispiel 1 der Patentanmeldung hergestellt wurden. Die elektronenmikroskopische Aufnahme wurde mit Hilfe der Negative-Staining-Technik hergestellt (Tickner, A.L., et al. (1990) Micr. Micros. Acta, 21, Seiten 91-99). Der Vergrößerungsfaktor der Abbildungen ist 50 000, die eingezeichneten Maßstäbe entsprechen 100 bzw. 200 nm. Man erkennt auf den Abbildungen sehr deutlich die für unilamellare Liposomen typischen Strukturen, d.h. einen wäßrigen, auf der Abbildung dunkel gefärbten Kern, umgeben von einem Phospholipidbilayer, das in der Abbildung als weißer Ring erscheint.

### Beispiel 2

### Herstellung von Enfluran-haltigen Liposomen

### a) Vordispergierung

10 g Enfluran werden in einen auf 4 °C abgekühlten, mit Stickstoff begasten und verschlossenen Laborreaktor gegeben. Dazu werden 20 g eines Ei-Phospholipid-Gemisches (Zusammensetzung: siehe Beispiel 1) gegeben und unter Rühren (20 U/min) aufgelöst. Es entsteht eine hochviskose Masse. Dann wird eine auf 4 °C abgekühlte Dispersion aus 20 g des gleichen Ei-phospholipid-Gemisches in 150 ml Hydratisierungsmedium (physiologische Kochsalzlösung) zugegeben und unter den in 1a) angegebenen Bedingungen 30 min. lang homogenisiert. Nach Beendigung der Homogenisierung wird, analog zu 1a), mit Hydratisierungsmedium auf ein Volumen von 200 ml aufgefüllt.

### b) Extrusion

Die in 2a) hergestellte Dispersion wird in einem Extruder (Edelstahlgehäuse; Innendurchmesser 8,2 cm; Außendurchmesser 12,2 cm; Höhe 9,3 cm; Drucklimit 40 bar), der mittels eines Wasserbades auf 4 °C abgekühlt ist, eingefüllt und mit Stickstoff bei 25 bar jeweils fünfmal durch Polycarbonat-Membranen (Fa. Nuclepore) mit den Porenweiten 400, 200 und 100 nm extrudiert.

Nach Sterilfiltration der Liposomendispersion wurden die folgenden Analysenergebnisse erhalten:

| | |
|---|---|
| Aussehen, Färbung: | opaleszent, gelblich |
| Teilchengröße: | 115 nm |
| Polydispersität: | 0,101 |
| Enflurangehalt: | 28,7 mg/ml |

### Beispiel 3

### Herstellung von Methoxyfluran-haltigen Liposomen

### a) Vordispergierung

80 g eines Soja-Phospholipid-Gemisches (Hauptkomponenten: PC 50 bis 100 %, PE 0 bis 40 %, Phasenübergangstemperatur ca. -20 °C), 20 g Methoxylfuran, 6 g Linolsäureethylester und 4 g Natriumoleat werden in physiologischer Kochsalzlösung zu 400 ml Dispersion gemäß 1a) verarbeitet.

### b) Hochdruckhomogenisierung

Die unter 3a) hergestellte Dispersion wird in das Vorratsgefäß eines Hochdruckhomogenisators mit LW-Ventilanordnung (z.B. Fa. Rannie) überführt und 20 min lang bei einem Druck von etwa 600 bar homogenisiert. Produktauslaß und Vorratsgefäß werden dabei mittels eines Wasserbades auf 4 °C temperiert. Nach Beendigung der Hochdruckhomogenisierung wird die Liposomendispersion sterilfiltriert.

Eine anschließend durchgeführte Analytik ergibt folgende Ergebnisse:

| | |
|---|---|
| Aussehen, Färbung: | opaleszent, bräunlich |
| Teilchengröße: | 85 nm |
| Polydispersität: | 0,2 |
| Methoxyflurangehalt: | 18,4 mg/ml |

### Beispiel 4

### Herstellung von Halothan-haltigen Liposomen

### a) Vordispergierung

20 g Halothan werden analog zu 2a) in einem gekühlten Laborreaktor vorgelegt, dazu werden 40 g eines Soja-Phospholipid-Gemisches (Zusammensetzung: siehe Beispiel 3) gegeben und unter Rühren (20 U/min) zu einer hochviskosen Masse verarbeitet. Parallel dazu wird eine Dispersion aus 40 g des gleichen Soja-Phospholipid-Gemisches, 6 g Linolsäureethylester und 4 g Natriumoleat in 300 ml Hydratisierungsmedium (10 mmol Tris HCl in 2,5 %iger wäßriger Glycerinlösung, pH 7,5) hergestellt. Wie in 2a) ausführlich beschrieben wird diese Dispersion mit dem Halothan/ Phospholipid-Gemisch vermischt und 30 min lang homogenisiert. Danach wird mit Hydratisierungsmedium auf ein Endvolumen von 400 ml aufgefüllt.

### b) Extrusion

Die Dispersion wird mittels einer Kolbenmembranpumpe mindestens 10 mal durch einen Keramikfilter (Ceraflo^{R}, Fa. Millipore) der Porenweite 0,2 »m mit einem Druck von 14 bar gepreßt. Das Vorratsgefäß wird dabei auf 4 °C gekühlt.

Die anschließende Analytik der Liposomendispersion erbrachte folgendes Ergebnis:

| | |
|---|---|
| Aussehen, Färbung: | opaleszent, hell |
| Teilchengröße: | 143 nm |
| Polydispersität: | 0,15 |
| Halothangehalt: | 24,3 mg/ml |

### Anwendungsbeispiel 1

### Durchführung einer Narkose durch intravenöse Injektion von Enfluran-Liposomen

Nach der Methode von Beispiel 2 wurden Enfluran-haltige Liposomen mit folgenden Charakteristika hergestellt:

| | |
|---|---|
| Aussehen, Färbung: | klar, gelblich |
| Teilchengröße: | 62,2 nm |
| Polydispersität: | 0,291 |
| Enflurangehalt: | 25,8 mg/ml |

Diese Liposomendispersion wurde in einer Bolusinjektion männlichen Wistarratten WU (Fa. Savo, Kisslegg, BRD) intravenös appliziert. Bei entsprechender Dosierung wurde bei den Tieren eine Narkose erzielt (siehe Tabelle):

| Tier Nr. | Injektionsvolumen (ml) | Dosis (mg/kgKG) | Schlafzeit (s) |
|---|---|---|---|
| 1 | 1 | 56,6 | -- |
| 2 | 1 | 63,7 | -- |
| 3 | 2 | 122,6 | > 10 |
| 4 | 2,5 | 152,9 | > 60 |

### Anwendungsbeispiel 2

### Durchführung einer Narkose durch intravenöse Injektion von Methoxyfluran-Liposomen

Nach der Methode von Beispiel 3 wurden Methoxyfluran-haltige Liposomen mit folgenden Charakteristika hergestellt:

| | |
|---|---|
| Aussehen, Färbung: | klar, hell |
| Teilchengröße: | 60,3 nm |
| Polydispersität: | 0,287 |
| Methoxyflurangehalt: | 35,5 mg/ml |

Diese Methoxyfluran-Liposomen wurden in einer Bolusinjektion Ratten der in Anwendungsbeispiel 1 beschriebenen Art intravenös appliziert. Bei einer angemessenen Dosierung konnte eine Narkose erzielt werden (siehe Tabelle):

| Tier Nr. | Injektionsvolumen (ml) | Dosis (mg/kgKG) | Schlafzeit (s) |
|---|---|---|---|
| 1 | 2 | 174,3 | exitus |
| 2 | 1 | 88,5 | > 60 |
| 3 | 1 | 84,0 | > 60 |

## Patentansprüche (Patentansprüche für folgende(n) Vertragsstaat(en): DE, FR, GB)

1. Flüchtige Inhalationsnarkotika enthaltende Liposomen, erhältlich durch
- Vordispergieren von 1 bis 40 Gew.-% wenigstens eines Phospholipids,
- 0,01 bis 10 Gew.-% wenigstens eines Inhalationsnarkotikums und
- 50 bis 98,99 Gew.-% eines Hydratisierungsmediums unter Bildung einer überwiegend multilamellare Vesikel enthaltenden Lipid-Wirkstoffdispersion,
- Extrudieren oder Homogenisieren unter Bildung überwiegend unilamellare Inhalationsnarkotika enthaltender Liposome und
- Sterilfiltern in an sich bekannter Weise.

2. Liposomen nach Anspruch 1, dadurch gekennzeichnet, daß sie als weitere Komponente bis zu 50 Gewichtsteile, bezogen auf 100 Gewichtsteile der Ausgangsmischung aus Phospholipid, Inhalationsnarkotikum und Hydratationsmedium, üblicher Hilfs- und Zusatzstoffe enthalten.

3. Liposomen nach Anspruch 1, dadurch gekennzeichnet, daß das Phospholipid ein einzelnes Phospholipid oder ein Phospholipidgemisch ist, das überwiegend aus Phospholipiden besteht, welches ausgewählt ist aus natürlichen, pflanzlichen oder tierischen Phospholipiden, halbsynthetischen oder synthetischen Phospholipiden.

4. Liposomen nach Anspruch 1, dadurch gekennzeichnet, daß das Phospholipid ein natürliches Phospholipid oder Phospholipidgemisch ist, das aus Soja oder Ei erhalten worden ist.

5. Liposomen nach Anspruch 1, dadurch gekennzeichnet, daß das Inhalationsnarkotikum ausgewählt ist aus fluorhaltigen Kohlenwasserstoffen und Ethern mit einem Siedepunkt von 48 bis 105 °C.

6. Liposomen nach Anspruch 1, dadurch gekennzeichnet, daß das Inhalationsnarkotikum ausgewählt ist aus Halothan, Methoxyfluran, Enfluran, Isofluran, Desfluran und/oder Sevofluran.

7. Liposomen nach Anspruch 1, dadurch gekennzeichnet, daß man als Hydratisierungsmedium eine physiologisch verträgliche isotone Lösung einsetzt.

8. Liposomen nach Anspruch 2, dadurch gekennzeichnet, daß man als übliche Hilfs- und Zusatzstoffe lipidartige Substanzen und/oder Antioxidantien und/oder Stoffe zur Stabilisierung der Bilayer einsetzt.

9. Liposomen nach Anspruch 8, dadurch gekennzeichnet, daß man als lipidartige Substanz einen C₄-C₂₀-Alkyl- oder Alken(poly)ylsäure-C₁-C₄-alkylester, und/oder C₄-C₂₀-Alkyl- oder Alken(poly)ylcarbonsäure, bzw. deren Salze, als Antioxidans Tocopherol, dessen Ester oder Ascorbylpalmitat und als Stoff zur Stabilisierung der Bilayer Cholesterin oder dessen Derivate einsetzt.

10. Verfahren zur Herstellung von flüchtige Inhalationsnarkotika enthaltenden Liposomen durch
- Vordispergieren von 1 bis 40 Gew.-% wenigstens eines Phospholipids,
- 0,01 bis 10 Gew.-% wenigstens eines Inhalationsnarkotikums und
- 50 bis 98,99 Gew.-% eines Hydratisierungsmediums unter Bildung einer überwiegend multilamellare Vesikel enthaltenden Lipid-Wirkstoffdispersion,
- Extrudieren oder Homogenisieren unter Bildung von überwiegend unilamellaren Inhalationsnarkotika enthaltenden Liposomen und
- anschließende Sterilfiltration in an sich bekannter Weise.

11. Verfahren nach Anspruch 10, dadurch gekennzeichnet, daß als weitere Komponenten bi szu 50 Gewichtsteile, bezogen auf 100 Gewichtsteile der Ausgangsmischung aus Phospholipid, Inhalationsnarkotikum und Hydratisierungsmedium, üblicher Hilfs- und Zusatzstoffe eingesetzt werden.

12. Verfahren nach Anspruch 10 oder 11, dadurch gekennzeichnet, daß man das Vordispergieren durchführt, indem man entweder das oder die Phospholipid(e) und gegebenenfalls die üblichen Hilfs- und Zusatzstoffe zuerst im Inhalationsnarkotikum auflöst und dann im Hydratisierungsmedium dispergiert, oder das oder die Phospholipid(e) und die üblichen Hilfs- und Zusatzstoffe im Hydratisierungsmedium dispergiert und dann das Inhalationsmedium hinzufügt.

13. Verfahren nach Anspruch 10 oder 12, dadurch gekennzeichnet, daß man das Vordispergieren unter Kühlung in einem gasdichten, abgeschlossenen Gefäß und unter Schutzgas durchführt.

14. Verwendung der Liposomen nach Ansprüchen 1 bis 9 oder hergestellt nach Ansprüchen 10 bis 13 zur Herstellung eines Arzneimittels zur Allgemeinanästhesie durch intravenöse Applikation.

15. Verwendung nach Anspruch 14 in Kombination mit anderen üblichen intravenösen Narkosemitteln wie Etomidat, Thiopental oder Propofol.

## Patentansprüche (Patentansprüche für folgende(n) Vertragsstaat(en): ES)

1. Verfahren zur Herstellung von flüchtige Inhalationsnarkotika enthaltenden Liposomen durch
- Vordispergieren von 1 bis 40 Gew.-% wenigstens eines Phospholipids,
- 0,01 bis 10 Gew.-% wenigstens eines Inhalationsnarkotikums und
- 50 bis 98,99 Gew.-% eines Hydratisierungsmediums unter Bildung einer überwiegend multilamellare Vesikel enthaltenden Lipid-Wirkstoffdispersion,
- Extrudieren oder Homogenisieren unter Bildung von überwiegend unilamellaren Inhalationsnarkotika enthaltenden Liposomen und
- anschließende Sterilfiltration in an sich bekannter Weise.

2. Verfahren nach Anspruch 1, dadurch gekennzeichnet, daß als weitere Komponente bis zu 50 Gewichtsteile, bezogen auf 100 Gewichtsteile der Ausgangsmischung aus Phospholipid, Inhalationsnarkotikum und Hydratisierungsmedium, üblicher Hilfs- und Zusatzstoffe hinzugefügt werden.

3. Verfahren nach Anspruch 1 oder 2, dadurch gekennzeichnet, daß man das Vordispergieren durchführt, indem man entweder das oder die Phospholipid(e) und gegebenenfalls die üblichen Hilfs- und Zusatzstoffe zuerst im Inhalationsnarkotikum auflöst und dann im Hydratisierungsmedium dispergiert, oder das oder die Phospholipid(e) und die üblichen Hilfs- und Zusatzstoffe im Hydratisierungsmedium dispergiert und dann das Inhalationsmedium hinzufügt.

4. Verfahren nach Anspruch 1 oder 3, dadurch gekennzeichnet, daß man das Vordispergieren unter Kühlung in einem gasdichten, abgeschlossenen Gefäß und unter Schutzgas durchführt.

5. Verfahren nach Anspruch 1, dadurch gekennzeichnet, daß das Phospholipid ein einzelnes Phospholipid oder, ein Phospholipidgemisch ist, das überwiegend aus Phospholipiden besteht, welches ausgewählt ist aus natürlichen, pflanzlichen oder tierischen Phospholipiden, halbsynthetischen oder synthetischen Phospholipiden.

6. Verfahren nach Anspruch 1, dadurch gekennzeichnet, daß das Phospholipid ein natürliches Phospholipid oder Phospholipidgemisch ist, das aus Soja oder Ei erhalten worden ist.

7. Verfahren nach Anspruch 1, dadurch gekennzeichnet, daß das Inhalationsnarkotikum ausgewählt ist aus fluorhaltigen Kohlenwasserstoffen und Ethern mit einem Siedepunkt von 48 bis 105 °C.

8. Verfahren nach Anspruch 1, dadurch gekennzeichnet, daß die Inhalationsnarkotika ausgewählt sind aus Halothan, Methoxyfluran, Enfluran, Isofluran, Desfluran und/oder Sevofluran.

9. Verfahren nach Anspruch 1, dadurch gekennzeichnet, daß man als Hydratisierungsmedium eine physiologisch verträgliche isotone Lösung einsetzt.

10. Verfahren nach Anspruch 2, dadurch gekennzeichnet, daß man als übliche Hilfs- und Zusatzstoffe lipidartige Substanzen und/oder Antioxidantien und/oder Stoffe zur Stabilisierung der Bilayer einsetzt.

11. Verfahren nach Anspruch 10, dadurch gekennzeichnet, daß man als lipidartige Substanz einen C₄-C₂₀-Alkyl- oder Alken(poly)ylsäure-C₁-C₄-alkylester, und/oder C₄-C₂₀-Alkyl- oder Alken(poly)ylcarbonsäure, bzw. deren Salze, als Antioxidans Tocopherol, dessen Ester oder Ascorbylpalmitat und als Stoff zur Stabilisierung der Bilayer Cholesterin oder dessen Derivate einsetzt.

## Claims (Claims for the following Contracting State(s): DE, FR, GB)

1. Liposomes containing volatile inhalation narcotics, obtainable by
- preliminarily dispersing
from 1 to 40% by weight of a phospholipid,
from 0.01 to 10% by weight of at least one inhalation narcotic, and
from 50 to 98.99% by weight of a hydrating medium to form a lipid-active substance dispersion which contains predominantly multi-lamellar vesicles,
- extrusion or homogenization to form predominantly unilaminar liposomes which contain inhalation narcotics, and
- sterile filtration in a *per se* known manner.

2. The liposomes according to claim 1, characterized in that they contain up to 50 parts by weight of conventional auxiliary materials and additives, as a further component, relative to 100 parts by weight of the initial mixture comprising phospholipid, inhalation narcotic and hydrating medium.

3. The liposomes according to claim 1, characterized in that the phospholipid is one single phospholipid or a phospholipid mixture predominantly consisting of phospholipids, which has been selected from natural vegetable or animal phospholipids, semi-synthetic or synthetic phospholipids.

4. The liposomes according to claim 1, characterized in that the phospholipid is a natural phospholipid or phospholipid mixture obtained from soybean or egg.

5. The liposomes according to claim 1, characterized in that the inhalation narcotic has been selected from fluorine-containing hydrocarbons and ethers having a boiling point of from 48 °C to 105 °C.

6. The liposomes according to claim 1, characterized in that the inhalation narcotic has been selected from halothane, methoxyflurane, enflurane, isoflurane, desflurane and/or sevoflurane.

7. The liposomes according to claim 1, characterized in that the hydrating medium employed is a physiologically compatible isotonic solution.

8. The liposomes according to claim 2, characterized in that lipid-like substances and/or antioxidants and/or materials for stabilizing the bilayer are employed as the conventional auxiliary materials and additives.

9. The liposomes according to claim 8, characterized in that a C₄-C₂₀-alkyl or -alken(poly)yl acid C₁-C₄-alkyl ester and/or a C₄-C₂₀-alkyl or -alken(poly)ylcarboxylic acid or its salts has been employed as the lipid-like substance, tocopherol, its ester or ascorbyl palmitate has been used as the antioxidant, and cholesterol or its derivative has been used as the material for stabilizing the bilayer.

10. A process for preparing liposomes containing volatile inhalation narcotics by
- preliminarily dispersing
from 1 to 40% by weight of a phospholipid,
from 0.01 to 10% by weight of at least one inhalation narcotic, and
from 50 to 98.99% by weight of a hydrating medium to form a lipid-active substance dispersion which contains predominantly multi-lamellar vesicles,
- extrusion or homogenization to form predominantly unilaminar liposomes which contain inhalation narcotics, and
- sterile filtration in a *per se* known manner.

11. The process according to claim 10, characterized in that up to 50 parts by weight of conventional auxiliary materials and additives are employed as a further component, relative to 100 parts by weight of the initial mixture comprising phospholipid, inhalation narcotic and hydrating medium.

12. The process according to claim 10 or 11, characterized in that the step of preliminary dispersing is carried out by
either first dissolving the phospholipid(s) and optionally the conventional auxiliary materials and additives in the inhalation narcotic followed by dispersing in the hydrating medium
or dispersing the phospholipid(s) and optionally the conventional auxiliary materials and additives in the hydrating medium and then adding the inhalation narcotic.

13. The process according to claim 10 or 12, characterized in that the step of preliminary dispersing is carried out in a gas-tight sealed vessel with cooling and under a protective gas.

14. Use of the liposomes according to claims 1 through 9 or prepared according to claims 10 to 13 for making a medicament for general anaesthesia by intravenous application.

15. The use according to claim 14 in combination with other conventional intravenous anaesthetics such as etomidate, thiopental or propofol.

## Claims (Claims for the following Contracting State(s): ES)

1. A process for preparing liposomes containing volatile inhalation narcotics by
- preliminarily dispersing
from 1 to 40% by weight of a phospholipid,
from 0.01 to 10% by weight of at least one inhalation narcotic, and
from 50 to 98.99% by weight of a hydrating medium to form a lipid-active substance dispersion which contains predominantly multi-lamellar vesicles,
- extrusion or homogenization to form predominantly unilaminar liposomes which contain inhalation narcotics, and
- sterile filtration in a *per se* known manner.

2. The process according to claim 1, characterized in that up to 50 parts by weight of conventional auxiliary materials and additives are employed as a further component, relative to 100 parts by weight of the initial mixture comprising phospholipid, inhalation narcotic and hydrating medium.

3. The process according to claim 1 or 2, characterized in that the step of preliminary dispersing is carried out by
either first dissolving the phospholipid(s) and optionally the conventional auxiliary materials and additives in the inhalation narcotic followed by dispersing in the hydrating medium
or dispersing the phospholipid(s) and optionally the conventional auxiliary materials and additives in the hydrating medium and then adding the inhalation narcotic.

4. The process according to claim 1 or 3, characterized in that the step of preliminary dispersing is carried out in a gas-tight sealed vessel with cooling and under a protective gas.

5. The process according to claim 1, characterized in that the phospholipid is one single phospholipid or a phospholipid mixture predominantly consisting of phospholipids, which has been selected from natural vegetable or animal phospholipids, semi-synthetic or synthetic phospholipids.

6. The process according to claim 1, characterized in that the phospholipid is a natural phospholipid or phospholipid mixture obtained from soybean or egg.

7. The process according to claim 1, characterized in that the inhalation narcotic has been selected from fluorine-containing hydrocarbons and ethers having a boiling point of from 48 °C to 105 °C.

8. The process according to claim 1, characterized in that the inhalation narcotic has been selected from halothane, methoxyflurane, enflurane, isoflurane, desflurane and/or sevoflurane.

9. The process according to claim 1, characterized in that the hydrating medium employed is a physiologically compatible isotonic solution.

10. The process according to claim 2, characterized in that lipid-like substances and/or antioxidants and/or materials for stabilizing the bilayer are employed as the conventional auxiliary materials and additives.

11. The process according to claim 10, characterized in that a C₄-C₂₀-alkyl or -alken(poly)yl acid C₁-C₄-alkyl ester and/or a C₄-C₂₀-alkyl or -alken(poly)ylcarboxylic acid or its salts has been employed as the lipid-like substance, tocopherol, its ester or ascorbyl palmitate has been used as the antioxidant, and cholesterol or its derivative has been used as the material for stabilizing the bilayer.

## Revendications (Revendications pour l'(les) Etat(s) contractant(s) suivant(s): DE, FR, GB)

1. Liposomes, contenant des anesthésiques d'inhalation volatils,que l'on peut obtenir par les étapes consistant :
- à prédisperser de 1 à 40 % en poids d'au moins un phospholipide,
- de 0,01 à 10 % en poids d'au moins un anesthésique d'inhalation et
- de 50 à 98,99 % en poids d'un milieu d'hydratation, avec formation d'une dispersion lipides-principe actif contenant des vésicules essentiellement multilamellaires,
- à procéder à une extrusion ou à une homogénéisation, avec formation de liposomes essentiellement unilamellaires, contenant des anesthésiques d'inhalation, et
- à procéder à une filtration stérile d'une manière connue en soi.

2. Liposomes selon la revendication 1, caractérisés en ce qu'ils contiennent comme autre constituant jusqu'à 50 parties en poids, pour 100 parties en poids du mélange de départ de phospholipide, d'anesthésique d'inhalation et de milieu d'hydratation, d'adjuvants et additifs usuels.

3. Liposomes selon la revendication 1, caractérisés en ce que le phospholipide est un phospholipide unique ou un mélange de phospholipides, qui est constitué essentiellement de phospholipides, qui est choisi parmi les phospholipides naturels, végétaux ou animaux, ou les phospholipides semi-synthétiques ou synthétiques.

4. Liposomes selon la revendication 1, caractérisés en ce que le phospholipide est un phospholipide naturel ou un mélange de phospholipides obtenu à partir de soja ou d'oeufs.

5. Liposomes selon la revendication 1, caractérisés en ce que l'anesthésique d'inhalation est choisi parmi les hydrocarbures fluorés et les éthers ayant un point d'ébullition de 48 à 105°C.

6. Liposomes selon la revendication 1, caractérisés en ce que l'anesthésique d'inhalation est choisi parmi l'halothane, le méthoxyflurane, l'enflurane, l'isoflurane, le desflurane et/ou le sévoflurane.

7. Liposomes selon la revendication 1, caractérisés en ce qu'on utilise comme milieu d'hydratation une solution isotonique physiologiquement tolérée.

8. Liposomes selon la revendication 2, caractérisés en ce qu'on utilise comme adjuvants et additifs usuels des substances lipidiques et/ou des anti-oxydants et/ou des substances destinées à stabiliser la bicouche.

9. Liposomes selon la revendication 8, caractérisés en ce qu'on utilise comme substance lipidique un ester alkylique en C₁-C₄ d'un acide alkylique ou alcène(poly)ylique en C₄-C₂₀, et/ou un acide [alkyl- ou alcène(poly)yl- en C₄-C₂₀] carboxylique, ou leurs sels, que l'on utilise comme anti-oxydant le tocophérol, ses esters ou du palmitate d'ascorbyle, et que l'on utilise comme substance destinée à stabiliser la bicouche du cholestérol ou ses dérivés.

10. Procédé pour préparer des liposomes contenant des anesthésiques d'inhalation volatils, qui consiste
- à prédisperser de 1 à 40 % en poids d'au moins un phospholipide,
- de 0,01 à 10 % en poids d'au moins un anesthésique d'inhalation, et
- de 50 à 98,99 % en poids d'un milieu d'hydratation, avec formation d'une dispersion lipide-principe actif contenant des vésicules essentiellement multilamellaires,
- à procéder à une extrusion ou à une homogénéisation avec formation de liposomes contenant des anesthésiques d'inhalation essentiellement unilamellaires, puis
- à procéder à une filtration stérile d'une manière connue en soi.

11. Procédé selon la revendication 10, caractérisé en ce qu'on utilise comme autres constituants jusqu'à 50% en poids, par rapport à 100 parties en poids du mélange de départ du phospholipide, de l'anesthésique d'inhala-tion et du milieu d'hydratation, d'adjuvants et d'addi-tifs usuels.

12. Procédé selon la revendication 10 ou 11, caractérisé en ce qu'on met en oeuvre la prédispersion en dissolvant d'abord dans l'anesthésique d'inhalation le ou les phospholipides et éventuellement les additifs et adjuvants usuels, puis en les dispersant dans le milieu d'hydratation, ou encore en dispersant dans le milieu d'hydratation le ou les phospholipides et les adjuvants et additifs usuels, puis en ajoutant le milieu d'inhalation.

13. Procédé selon la revendication 10 ou 12, caractérisé en ce qu'on procède à la prédispersion sous refroidissement dans un récipient fermé étanche aux gaz, sous atmosphère d'un gaz protecteur.

14. Utilisation des liposomes selon les revendications 1 à 9, ou préparés selon les revendications 10 à 13, pour préparer un médicament destiné à l'anesthésie générale, par administration intraveineuse.

15. Utilisation selon la revendication 14 en combinaison avec d'autres anesthésiques intraveineux usuels tels que l'étomidate, le thiopental ou le propofol.

## Revendications (Revendications pour l'(les) Etat(s) contractant(s) suivant(s): ES)

1. Procédé pour préparer des liposomes contenant des anesthésiques d'inhalation volatils, qui consiste
- à prédisperser de 1 à 40 % en poids d'au moins un phospholipide,
- de 0,01 à 10 % en poids d'au moins un anesthésique d'inhalation, et
- de 50 à 98,99 % en poids d'un milieu d'hydratation, avec formation d'une dispersion lipide-principe actif contenant des vésicules essentiellement multilamellaires,
- à procéder à une extrusion ou à une homogénéisation avec formation de liposomes contenant des anesthésiques d'inhalation essentiellement unilamellaires, puis
- à procéder à une filtration stérile d'une manière connue en soi.

2. Procédé selon la revendication 1, caractérisé en ce qu'on ajoute comme autre constituant jusqu'à 50 parties en poids, pour 100 parties en poids du mélange de départ du phospholipide, de l'anesthésique d'inhalation et du milieu d'hydratation, d'adjuvants et d'additifs usuels.

3. Procédé selon la revendication 1 ou 2, caractérisé en ce qu'on met en oeuvre la prédispersion en dissolvant d'abord dans l'anesthésique d'inhalation le ou les phospholipides et éventuellement les additifs et adjuvants usuels, puis en les dispersant dans le milieu d'hydratation, ou encore en dispersant dans le milieu d'hydratation le ou les phospholipides et les adjuvants et additifs usuels, puis en ajoutant le milieu d'inhalation.

4. Procédé selon la revendication 1 ou 3, caractérisé en ce qu'on procède à la prédispersion sous refroidissement dans un récipient fermé étanche aux gaz, sous atmosphère d'un gaz protecteur.

5. Procédé selon la revendication 1, caractérisé en ce que le phospholipide est un phospholipide unique ou un mélange de phospholipides, qui est constitué essentiellement de phospholipides, qui est choisi parmi les phospholipides naturels, végétaux ou animaux, ou les phospholipides semi-synthétiques ou synthétiques.

6. Procédé selon la revendication 1, caractérisé en ce que le phospholipide est un phospholipide naturel ou un mélange de phospholipides obtenu à partir de soja ou d'oeufs.

7. Procédé selon la revendication 1, caractérisé en ce que l'anesthésique d'inhalation est choisi parmi les hydrocarbures fluorés et les éthers ayant un point d'ébullition de 48 à 105°C.

8. Procédé selon la revendication 1, caractérisé en ce que l'anesthésique d'inhalation est choisi parmi l'halothane, le méthoxyflurane, l'enflurane, l'isoflurane, le desflurane et/ou le sévoflurane.

9. Procédé selon la revendication 1, caractérisé en ce qu'on utilise comme milieu d'hydratation une solution isotonique physiologiquement tolérée.

10. Procédé selon la revendication 2, caractérisé en ce qu'on utilise comme adjuvants et additifs usuels des substances lipidiques et/ou des antioxydants et/ou des substances destinées à stabiliser la bicouche.

11. Procédé selon la revendication 10, caractérisé en ce qu'on utilise comme substance lipidique un ester alkylique en C₁-C₄ d'un acide alkylique ou alcène(poly)ylique en C₄-C₂₀, et/ou un acide [alkyl- ou alcène(poly)yl- en C₄-C₂₀] carboxylique, ou leurs sels, que l'on utilise comme anti-oxydant le tocophérol, ses esters ou du palmitate d'ascorbyle, et que l'on utilise comme substance destinée à stabiliser la bicouche du cholestérol ou ses dérivés.
